Europäisches Patentamt

(19) European Patent Office

Office européen des brevets

(11) Publication number: **0 032 784**
**B1**

(12) **EUROPEAN PATENT SPECIFICATION**

(45) Date of publication of patent specification: **18.04.84**    (51) Int. Cl.³: **C 07 C 143/38**

(21) Application number: **81300039.5**

(22) Date of filing: **07.01.81**

(54) Production of hydrazobenzene-3,3'-disulphonic acid.

<table>
<tr><td>

(30) Priority: **19.01.80 GB 8001846**

(43) Date of publication of application:
**29.07.81 Bulletin 81/30**

(45) Publication of the grant of the patent:
**18.04.84 Bulletin 84/16**

(84) Designated Contracting States:
**BE CH DE FR GB IT LI NL**

(56) References cited:
**US - A - 2 233 129**
**US - A - 3 063 980**
**US - A - 3 260 750**

**EUGEN MULLER: "Methoden der Organischen
Chemie (HOUBEN-WEYL) 4th Edition, vol. X/2:
"Stickstoffverbindungen I, Teil 2" 1967, Georg
Thieme Verlag, Stuttgart, DE 3.
"Hydrazoverbindungen aus Nitroverbindungen
durch katalytische Reduktion", pages 701-702.**

</td><td>

(73) Proprietor: **The Clayton Aniline Company Limited
P.O. Box 2,
Clayton Manchester M11 4AP (GB)**

(72) Inventor: **Hildreth, John David
Damson Cottage Andertons Lane Henbury
Macclesfield Cheshire (GB)**
Inventor: **Haslam, David Geoffrey
72, Church Road Kearsley
Bolton Lancashire BL4 8AP (GB)**

(74) Representative: **Sharman, Thomas
CIBA-GEIGY (UK) Limited Tenax Road
Trafford Park Manchester M17 1WT (GB)**

(56) References cited:
**CHEMICAL ABSTRACTS, vol. 38, no. 6, March
20, 1944, column 1214 4/5 Columbus, Ohio,
USA A.A. STREL'TSOVA et al. "Catalytic
reduction of nitro compounds of the aromatic
series. II."**

</td></tr>
</table>

Courier Press, Leamington Spa, England.

Production of hydrazobenzene-3,3'-disulphonic acid

The present invention relates to the production of hydrazobenzene-3,3'-disulphonic acid.

It is known that hydrazobenzene-3,3'-disulphonic acid may be produced by the reduction of 3-nitrobenzenesulphonic acid with zinc and aqueous sodium hydroxide. With this method, however, the use of zinc powder is expensive and furthermore an additional process is needed to remove zinc oxide formed as by-product. Further disadvantages are that a) the hydrazobenzene compound is oxidised to some extent during the filtration of the residual zinc oxide, to azo and azoxy compounds; b) that the yield is only 40—70% theoretical and c) that the reaction takes 30 to 40 hours. Other suggested methods of producing hydrazobenzene-3,3'-disulphonic acid include the reduction of the parent nitro compound with ferosilicon in sodium hydroxide (Kogyu Kagaku Zasshi CA 60 1334—6 (1957), CA 53 17021c and CA 52 8993d), and the reduction of nitrobenzene-3 sulphonic acid with formaldehyde in the presence of a naphthoquinonoid type catalyst in a nitrogen atmosphere (Japan Kokai 78 63,340). Alternatively, 1,1'-azoxybenzene-3,3'-disulphonic acid in an organic solvent may be reduced with a mixture of aqueous sodium hydrogen sulphide and sodium thiosulphate (W. German OLS 2,546,656).

Processes for the reduction of nitrobenzene to hydrazobenzene are known. United States Patent Specification No. 3063980 describes the reduction using a reducing mixture of an alkali metal hydroxide and a lower alcohol in the presence of at least 1/1000 of a mole of nickel or a noble metal catalyst. Houbel-Weyl: Methoden der organischen Chemie, 4 Aufl., Vol X/2 Stuttgart (1967) at pages 701—702 describes the reduction of nitrobenzene by catalytic hydrogenation using alcoholic alkali. In one reaction described a small amount of water is also present. Production of hydrazobenzene-dicarboxylic acids is also described by catalytic hydrogenation using hydrazine hydrate in the presence of alcoholic alkali, but in these cases no water is present during the reaction. The products are recovered by various techniques including neutralisation, filtration and recrystallisation.

We have now surprisingly found that hydrazobenzene-3,3'-disulphonic acid may be prepared by the catalytic hydrogenation of 3-nitrobenzene sulphonic acid under aqueous alkaline conditions, giving a product suitable for further use after removing the catalyst.

Accordingly, the present invention provides a process for the manufacture of hydrazobenzene-3,3'-disulphonic acid which comprises reacting 3-nitrobenzene-sulphonic acid with hydrogen in the presence of a Group VIII metal catalyst, a base, and sufficient water to dissolve the resulting hydrazobenzene-3-3'-disulphonic acid.

The 3-nitrobenzene sulphonic acid may be used in the form of the ammonium salt or as an alkali metal or alkaline earth metal salt, such as the sodium, potassium, calcium or barium salt, but preferably as the sodium or potassium salt.

The catalyst may be any Group VIII metal that is known to be suitable for carrying out catalytic hydrogenation reactions, such as nickel, platinum, palladium, ruthenium and rhodium. It is preferred to use palladium on charcoal as the catalyst in suitable catalytic amounts. The amount of catalyst may be varied over a wide range and may be from 0.005% to 0.1% metal based on the weight of the nitro compound. It is preferred to use about 0.01%.

The reaction may be carried out under elevated hydrogen pressure which may be from 1 to 100 bar, preferably from 5 to 30 bar. The temperature of reaction may be from ambient to 100°C, preferably between 60° and 85°C. At higher temperatures losses in yield occur.

The reaction is continued until consumption of hydrogen ceases which may take from 1 to 10 hours. The time taken is dependant on the catalyst concentration, the hydrogen pressure, the temperature of the reaction, and on the efficiency of the mixing during the reaction. For example, reaction times may be reduced by carrying out the hydrogenation in a spray nozzle high circulation reactor, such as a Buss loop reactor. Such a loop reactor normally renders an enhanced selectivity to the catalyst through more efficient mixing and cooling of the exothermic reaction. After uptake of hydrogen ceases, it is preferred to maintain the reaction conditions for an additional time, e.g. up to 30 minutes, preferably about 15 minutes to ensure that the reaction is complete.

Before the hydrogenation is started, the reactor should be purged to remove oxygen. The purging can be carried out using an inert gas, such as nitrogen or, preferably, hydrogen.

The amount of water used should be sufficient to dissolve the resulting hydrazobenzene-3,3'-disulphonic acid. The actual quantity of water needed for this will depend on the purity of the starting material, on the particular 2-nitrobenzene sulphonic acid salt and on the base used.

The base may be ammonium hydroxide or carbonate or an alkali metal or alkaline earth metal hydroxide or carbonate. Preferably the base is an alkali metal hydroxide, such as sodium or potassium hydroxide, but sodium hydroxide is most preferred. The equivalent ratio of base to 3-nitrobenzene sulphonic acid may vary from 4:1 to 0.5:1, but is preferably about 1:1.

The resulting hydrazobenzene-3,3'-disulphonic acid can be easily separated from the catalyst by filtering. The product may then be converted into benzidine-2,2'-disulphonic acid by known processes. This compound may then be used as a starting material for the manufacture of dyestuffs and pigments by diazotisation, followed by coupling with various coupling components.

The invention is illustrated by the following Examples, in which parts by weight bear the same relationship to parts by volume as do kilograms to litres.

## Example 1

A shaking autoclave was charged with 71.2 parts by weight potassium 3-nitrobenzene-sulphonate. To this was added 220 parts by weight water, 25.2 parts by weight aqueous sodium hydroxide 47% by weight, and 0.15 parts by weight of catalyst. The catalyst comprised 5% palladium on carbon, and was supplied as a 50% water wet paste. The reactor was then closed, purged twice with hydrogen and then with agitation heated to 80—85°C. At 80—85°C hydrogen was introduced at 10 bar and with good agitation the temperature was maintained for 2 hours when consumption of hydrogen ceased. The reaction conditions were maintained for a further 15 minutes to ensure the reaction was complete.

The pressure was then released, and the catalyst filtered maintaining an inert gas atmosphere.

The solution of hydrazobenzene-3,3'-disulphonic acid was then re-arranged by known processes to yield after drying 43.4 parts by weight white benzidine 2,2'-disulphonic acid, i.e. 81.1% theoretical yield.

## Example 2

The apparatus was the same as for Example 1. To this was added 66.5 parts by weight sodium 3-nitrobenzenesulphonate, 120 parts by weight water, 25.2 parts by weight aqueous sodium hydroxide 47% by weight, and 0.15 parts by weight of catalyst as used in Example 1. The catalytic reduction was performed as per Example 1 to give, finally, 42.7 parts by weight of white benzidine 2,2'-disulphonic acid. The yield was 77% theoretical.

## Claims

1. A process for the manufacture of hydrazobenzene-3,3'-disulphonic acid by reduction of 3-nitrobenzene sulphonic acid characterised in that the reduction is effected with hydrogen in the presence of a Group VIII metal catalyst, a base and sufficient water to dissolve the resulting hydrazobenzene-3,3'-disulphonic acid.

2. A process as claimed in Claim 1, characterised in that the 3-nitrobenzene sulphonic acid is used in the form of an alkali metal or alkaline earth metal salt, or as the ammonium salt.

3. A process as claimed in Claim 1 or 2, characterised in that the Group VIII metal catalyst is nickel, platinum, palladium, ruthenium or rhodium.

4. A process as claimed in any preceding claim, characterised in that the catalyst is palladium on charcoal.

5. A process as claimed in any preceding claim, characterised in that the amount of catalyst is from 0.005% to 0.1% metal based on the weight of nitro compound.

6. A process as claimed in any preceding claim, characterised in that it is carried out at a pressure of 1 to 100 bar.

7. A process as claimed in any preceding claim, characterised in that it is carried out at a temperature from ambient to 100°C.

8. A process as claimed in any preceding claim, characterised in that the base is ammonium hydroxide or carbonate or an alkali metal or alkaline earth metal hydroxide or carbonate.

9. A process as claimed in any preceding claim, characterised in that the equivalent ratio of base to 3-nitrobenzene sulphonic acid is from 4:1 to 0.5:1.

## Revendications

1. Procédé de fabrication d'acide hydrazobenzène-3,3'-disulfonique par réduction d'acide 3-nitrobenzène-sulfonique, caractérisé en ce qu'on effectue la réduction avec de l'hydrogène en présence d'un catalyseur d'un métal du groupe VIII, d'une base et d'une quantité d'eau suffisante pour dissoudre l'acide hydrazobenzène-3,3'-disulfonique formé.

2. Procédé suivant la revendication 1, caractérisé en ce que l'acide 3-nitrobenzène-sulfonique est utilisé sous forme d'un sel d'un métal alcalin ou d'un métal alcalino-terreux ou encore sous forme de son sel d'ammonium.

3. Procédé suivant la revendication 1 ou 2, caractérisé en ce que le catalyseur du métal du groupe VIII est le nickel, le platine, le palladium, le ruthénium ou le rhodium.

4. Procédé suivant l'une quelconque des revendications précédentes, caractérisé en ce que le catalyseur est du charbon palladié.

5. Procédé suivant l'une quelconque des revendications précédentes, caractérisé en ce que la quantité de catalyseur se situe entre 0,005% et 0,1% de métal, calculé sur le poids du nitro-composé.

6. Procédé suivant l'une quelconque des revendications précédentes, caractérisé en ce qu'on l'effectue sous une pression de 1 à 100 bars.

7. Procédé suivant l'une quelconque des revendications précédentes, caractérisé en ce qu'on l'effectue à une température se situant entre la température ambiante et 100°C.

8. Procédé suivant l'une quelconque des revendications précédentes, caractérisé en ce que la base est l'hydroxyde ou le carbonate d'ammonium ou un hydroxyde ou un carbonate d'un métal alcalin ou d'un métal alcalino-terreux.

9. Procédé suivant l'une quelconque des revendications précédentes, caractérisé en ce que le rapport équivalent entre la base et l'acide 3-nitrobenzène-sulfonique est de 4:1 a 0,5:1.

**Patentansprüche**

1. Verfahren zur Herstellung von Hydrazobenzol-3,3'-disulfonsäure durch Reduktion von 3-Nitrobenzolsulfonsäure, dadurch gekennzeichnet, dass die Reduktion mit Wasserstoff in Gegenwart eines Metallkatalysators aus Gruppe VIII, einer Base und genügend Wasser zur Auflösung der entstandenen Hydrazobenzol-3,3'-disulfonsäure erfolgt.

2. Verfahren nach Anspruch 1, dadurch gekennzeichnet, dass die 3-Nitrobenzolsulfonsäure in Form eines Alkali- oder Erdalkalisalzes oder als Ammoniumsalz eingesetzt wird.

3. Verfahren nach Anspruch 1 oder 2, dadurch gekennzeichnet, dass als Metallkatalysator aus Gruppe VIII Nickel, Platin, Palladium, Ruthenium oder Rhodium vorliegt.

4. Verfahren nach einem der vorhergehenden Ansprüche, dadurch gekennzeichnet, dass als Katalysator Palladiumkohle vorliegt.

5. Verfahren nach einem der vorhergehenden Ansprüche, dadurch gekennzeichnet, dass die Katalysatormenge 0,005% bis 0,1% Metall, bezogen auf das Gesamtgewicht der Nitroverbindung, beträgt.

6. Verfahren nach einem der vorhergehenden Ansprüche, dadurch gekennzeichnet, dass man es unter einem Druck von 1 bis 100 bar durchführt.

7. Verfahren nach einem der vorhergehenden Ansprüche, dadurch gekennzeichnet, dass man es bei einer Temperatur zwischen Raumtemperatur und 100°C durchführt.

8. Verfahren nach einem der vorhergehenden Ansprüche, dadurch gekennzeichnet, dass als Base Ammoniumhydroxyd oder -carbonat oder ein Alkali- bzw. Erdalkalihydroxyd bzw. -carbonat vorliegt.

9. Verfahren nach einem der vorhergehenden Ansprüche, dadurch gekennzeichnet, dass das Aequivalentverhältnis der Base zu 3-Nitrobenzolsulfonsäure 4:1 bis 0,5:1 beträgt.